# EUROPEAN PATENT APPLICATION

(11) **EP 4 151 653 A1**
(43) Date of publication of application: **22.03.2023**
(21) Application number: 21788934.4
(22) Date of filing: 01.04.2021
(51) Int. Cl.: C07K 16/28, C12N 15/13, C07K 19/00, C12N 15/62, C12N 15/867, C12N 5/10, A61P 35/00

(54) **CD22-TARGETED CHIMERIC ANTIGEN RECEPTOR, PREPARATION METHOD THEREFOR AND APPLICATION THEREOF**

(30) Priority: 16.04.2020 CN 202010301357
(71) Applicant: Shanghai Cellular Biopharmaceutical Group Ltd., Shanghai, 201210 (CN); Wuxi Cellular Biopharmaceutical Group Ltd., Wuxi, Jiangsu 214174 (CN)
(72) Inventor: YAO, Yihong, Shanghai 201210 (CN); ZHU, Lin, Shanghai 201210 (CN); LI, Yanfeng, Shanghai 201210 (CN); WEI, Yutian, Shanghai 201210 (CN); YAO, Xin, Shanghai 201210 (CN); ZHU, Shigui, Shanghai 201210 (CN); HUANG, Jiaqi, Shanghai 201210 (CN); ZHANG, Li, Shanghai 201210 (CN)
(74) Representative: Cabinet Becker et Associés
(86) International application number: PCT/CN2021/085062
(87) International publication number: WO 2021/208750

(57) **Abstract**

Provided are a CD22-targeted chimeric antigen receptor, a preparation method therefor and an application thereof. The chimeric antigen receptor comprises a leader sequence, a CD22-targeted scFv, a hinge region, a transmembrane region, and an intracellular signal domain. Provided are a nucleic acid molecule encoding the chimeric antigen receptor and a corresponding expression vector, a CAR-T cell, and an application thereof. The chimeric antigen receptor targets CD22 positive cells and can be used for treating CD22-positive B-cell leukemia, and some CD19-negative and CD22-positive patients in which acute B-cell leukemia has recurred after anti-CD19 CAR-T treatment.

## Description

### Technical field

The present invention relates to the field of biomedicine, and more particularly to a CD22-targeted chimeric antigen receptor, a preparation method therefor and an application thereof.

### Background technology

Hematological malignancies account for approximately 10% of human malignancies, and 95% of hematological malignancies are derived from B lymphocytes. Conventional chemotherapy and radiotherapy play an important role in the treatment of hematological malignancies, but conventional cancer treatment methods have encountered bottlenecks, and surgery, radiotherapy and chemotherapy have poor specificity, obvious side effects, and high recurrence and metastasis. Although some patients have significant responses, curing most has proven difficult. Therefore, more effective new treatment methods have always been the focus of exploration in this field.

The focus of cancer treatment has turned to the most promising tumor immunotherapy. So far, two CD19-targeted chimeric antigen receptor T-cell therapy (CAR-T) products have been approved and marketed abroad for the treatment of acute lymphoblastic leukemia in children and young adult patients and second-line or multi-line systemic therapy for recurrent or refractory large B-cell lymphoma in adults. However, after anti-CD19 CAR-T treatment, 40% of patients have relapsed after achieving complete remission at 1 month of treatment, and more than 60% of the relapsed patients have shown CD19-negative tumor cell escape. In some patients, the expression of CD19 in tumor cells is restricted, and no response or drug resistance is often observed when they receive anti-CD19 CAR-T cell therapy. Therefore, it is urgent to screen CAR-T structures targeting B-cell leukemia-related antigens other than CD19 to treat patients with malignant lymphomas.

Although anti-CD19 CAR-T has outstanding efficacy, it is ineffective for some leukemia patients with missing CD19 antigen expression in B cells; some patients have decreased or even lost the expression of CD19 antigen after anti-CD19 CAR-T treatment, resulting in unsatisfactory treatment outcomes and easy relapse with continued persistent expression of CD22.

Therefore, there is an urgent need in the field to develop new and effective drugs for the treatment of tumors.

### Summary of the invention

The purpose of the present invention is to provide a CD22-targeted chimeric antigen receptor, a preparation method therefor and an application thereof.

Specifically, the purpose of the present invention is to provide a sequence of a CD22-targeted chimeric antigen receptor, a method for preparing T cells modified thereby (CART-CD22) and an activity assay thereof. The present invention provides a chimeric antigen receptor structure for the treatment of CD22-positive B-cell lymphomas.

In a first aspect of the present invention, a chimeric antigen receptor (CAR) is provided, the antigen binding domain (i.e., scFv) of the chimeric antigen receptor comprises an antibody heavy chain variable region shown in SEQ ID NO.: 1 or 3 and an antibody light chain variable region shown in SEQ ID NO.: 2 or 4.

In another preferred example, the antigen heavy chain variable region and the antigen light chain variable region are connected by a linking peptide.

In another preferred example, the structure of the antigen binding domain is shown in formula I or II below:

V_{L}-V_{H} (I); V_{H}-V_{L} (II)

In the formula, V_{H} is the antigen heavy chain variable region; V_{L} is the antigen light chain variable region; "-" is a linking peptide or peptide bond.

In another preferred example, the structure of the antigen binding domain is shown in formula I.

In another preferred example, the amino acid sequence of the V_{L} is shown in SEQ ID NO.: 1, and the amino acid sequence of the V_{H} is shown in SEQ ID NO.: 2. (Note: CAR-T22.13)

In another preferred example, the amino acid sequence of the V_{L} is shown in SEQ ID NO.: 3, and the amino acid sequence of the V_{H} is shown in SEQ ID NO.: 4. (Note: CAR-T22.14)

In another preferred example, the amino acid sequence of the linking peptide is shown in SEQ ID NO.: 16.

In another preferred example, the antigen binding domain binds to CD22, preferably to human CD22.

In another preferred example, the heavy chain variable region and the light chain variable region of the antigen binding domain are derived from a humanized or human antibody.

In another preferred example, the structure of the chimeric antigen receptor is shown in formula III below:

L-V_{L}-V_{H}-H-TM-C-CD3 ζ (III)

In the formula,
L is an optional leader sequence, i.e., a signal peptide;
H is a hinge region;
TM is the transmembrane domain;
C is a costimulatory signal molecule;
CD3 ζ is a cytoplasmic signaling sequence derived from CD3 ζ;
V_{H}, V_{L} and "-" are as defined above.

In another preferred example, the L is a signal peptide of a protein selected from the group of CD8, CD28, GM-CSF, CD4, and CD137, or a combination thereof.

In another preferred example, the L is a signal peptide derived from CD8.

In another preferred example, the amino acid sequence of the L is shown in SEQ ID NO.: 9.

In another preferred example, the H is a hinge region of a protein selected from the group of CD8, CD28, and CD137, or a combination thereof.

In another preferred example, the H is a hinge region derived from CD8α.

In another preferred example, the amino acid sequence of the H is shown in SEQ ID NO.: 10.

In another preferred example, the TM is a transmembrane region of a protein selected from the group of CD28, CD3 epsilon, CD45, CD4, CD5, CD8, CD9, CD16, CD22, CD33, CD37, CD64, CD80, CD86, CD134, CD137, and CD154, or a combination thereof.

In another preferred example, the TM is a transmembrane region derived from CD8α.

In another preferred example, the sequence of the TM is shown in SEQ ID NO.: 11.

In another preferred example, the C is a costimulatory signal molecule of a protein selected from the group of OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, and TLR2, or a combination thereof.

In another preferred example, the C is a costimulatory signal molecule derived from 4-1BB or CD28, preferably a costimulatory signal molecule derived from 4-1BB.

In another preferred example, the amino acid sequence of the C is shown in SEQ ID NO.: 12.

In another preferred example, the amino acid sequence of CD3 ζ is shown in SEQ ID NO.: 13.

In another preferred example, the amino acid sequence of the chimeric antigen receptor is shown in SEQ ID NO.: 14 or 15.

In a second aspect of the present invention, a nucleic acid molecule is provided, which encodes the chimeric antigen receptor (CAR) described in the first aspect of the present invention.

In another preferred example, the nucleic acid molecule is isolated.

In a third aspect of the present invention, a vector is provided, which contains the nucleic acid molecule described in the second aspect of the present invention.

In another preferred example, the vector is selected from the group of DNAs, RNAs, plasmids, lentiviral vectors, adenoviral vectors, adeno-associated viral vectors (AAVs), retroviral vectors, and transposons, or a combination thereof.

In another preferred example, the vector is selected from the group of plasmids and viral vectors.

In another preferred example, the vector is in the form of virus particles.

In another preferred example, the vector is a lentiviral vector.

In another preferred example, the vector comprises one or more promoters, which are operably linked to the nucleic acid sequence, enhancers, transcription termination signals, polyadenylation sequences, origins of replication, selectable markers, nucleic acid restriction sites, and/or homologous recombination sites.

In a fourth aspect of the present invention, a host cell is provided, which contains the vector described in the third aspect of the present invention or a chromosome integrated with an exogenous nucleic acid molecule as described in the second aspect of the present invention or expressing the CAR described in the first aspect of the present invention.

In another preferred example, the host cell is an isolated cell.

In another preferred example, the host cell is a genetically engineered cell.

In another preferred example, the host cell is a mammalian cell.

In another preferred example, the host cell is a T cell or an NK cell, preferably a T cell.

In another preferred example, the host cell is a CAR-T cell or a CAR-NK cell, preferably a CAR-T cell.

In a fifth aspect of the present invention, a method for preparing an engineered immune cell is provided, the immune cell expresses the CAR described in the first aspect of the present invention, and the method comprises the following steps:
(a) providing an immune cell to be engineered; and
(b) transducing the nucleic acid molecule described in the second aspect of the present invention or the vector described in the third aspect of the present invention into the immune cell to obtain the engineered immune cell.

In another preferred example, the engineered immune cell is a CAR-T cell or a CAR-NK cell.

In another preferred example, in step (a), the method further comprises culturing the immune cell to be engineered in a GT-551 serum-free medium containing 0.1-10% (preferably 0.5%-5%, more preferably 0.8%-2%) human albumin, wherein the content of human albumin is calculated based on the total weight of the medium.

In another preferred example, the method further comprises a step of testing the function and effectiveness of the obtained engineered immune cell.

In a sixth aspect of the present invention, a formulation is provided which contains the chimeric antigen receptor described in the first aspect of the present invention, the nucleic acid molecule described in the second aspect of the present invention, the vector described in the third aspect of the present invention, or the host cell described in the fourth aspect of the present invention, and a pharmaceutically acceptable carrier, diluent or excipient.

In another preferred example, the formulation is a liquid formulation.

In another preferred example, the dosage form of the formulation is an injection.

In another preferred example, the concentration of the CAR-T cells in the formulation is 1 × 10³-1 × 10⁸ cells/ml, preferably 1 × 10⁴-1 × 10⁷ cells/ml.

In a seventh aspect of the present invention, a use of the chimeric antigen receptor described in the first aspect of the present invention, the nucleic acid molecule described in the second aspect of the present invention, the vector described in the third aspect of the present invention, or the host cell described in the fourth aspect of the present invention, or the formulation described in the sixth aspect of the present invention is provided, the use being for preparing a drug or a preparation for preventing and/or treating cancers or tumors.

In another preferred example, the tumors are selected from the group of hematological tumors, and solid tumors, or a combination thereof.

In another preferred example, the hematological tumors are selected from the group of acute myeloid leukemia (AML), multiple myeloma (MM), chronic lymphocytic leukemia (CLL), acute lymphocytic leukemia (ALL), and diffuse large B-cell lymphoma (DLBCL), or a combination thereof.

In another preferred example, the solid tumors are selected from the group of gastric carcinomas, peritoneal metastasis of gastric carcinomas, hepatomas, leukemias, renal carcinomas, lung carcinomas, small intestine carcinomas, bone carcinomas, prostate carcinomas, colon and rectum carcinomas, breast carcinomas, colorectal carcinomas, cervical carcinomas, ovarian carcinomas, lymphomas, nasopharyngeal carcinomas, adrenal tumors, bladder tumors, non-small cell lung carcinomas (NSCLCs), gliomas, and endometrial carcinomas, or a combination thereof.

In another preferred example, the tumors are CD22-positive tumors, preferably CD22-positive B-cell lymphomas, multiple myelomas, or plasma cell leukemia.

In an eighth aspect of the present invention, a kit used for preparing the host cell described in the fourth aspect of the present invention is provided, which includes a container and the nucleic acid molecule described in the second aspect of the present invention or the vector described in the third aspect of the present invention located in the container.

In a ninth aspect of the present invention, a method for treating a disease is provided, comprising administering an appropriate amount of the vector described in the third aspect of the present invention, the host cell described in the fourth aspect of the present invention, or the formulation described in the sixth aspect of the present invention to a subject in need.

In another preferred example, the disease is a cancer or a tumor.

It should be understood that, within the scope of the present invention, the above technical features of the present invention and the technical features described in detail below (e.g., the embodiments) may be combined to form a new or preferred technical solution. Due to the space limitation, it will not be detailed here.

### Description of the drawings

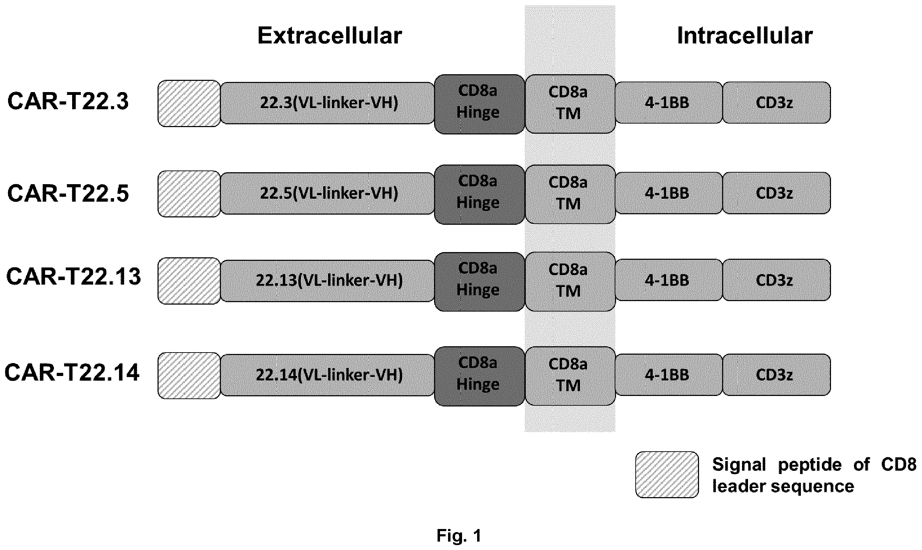

Figure 1 is a schematic structural diagram of a CD22-targeted chimeric antigen receptor. The structure of the CAR comprises a leader sequence, an antigen recognition sequence, a connecting region, a transmembrane region, a costimulater signal region and a CD3 zeta signaling region.

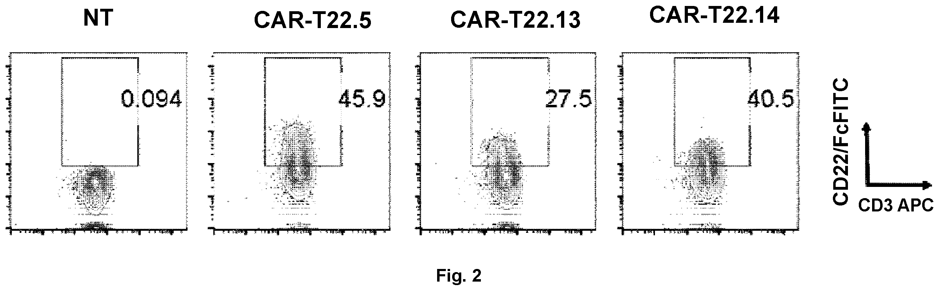

Figure 2 shows the measurement of the transfection efficiency of T cells engineered by a CD22-targeted chimeric antigen receptor. The expression level of CAR gene-encoded protein on the surface of T cell membranes in CART-CD22s cells was analyzed by flow cytometry at Day 6 of culturing through staining with the Fc fragment of a recombinant human CD22 protein.

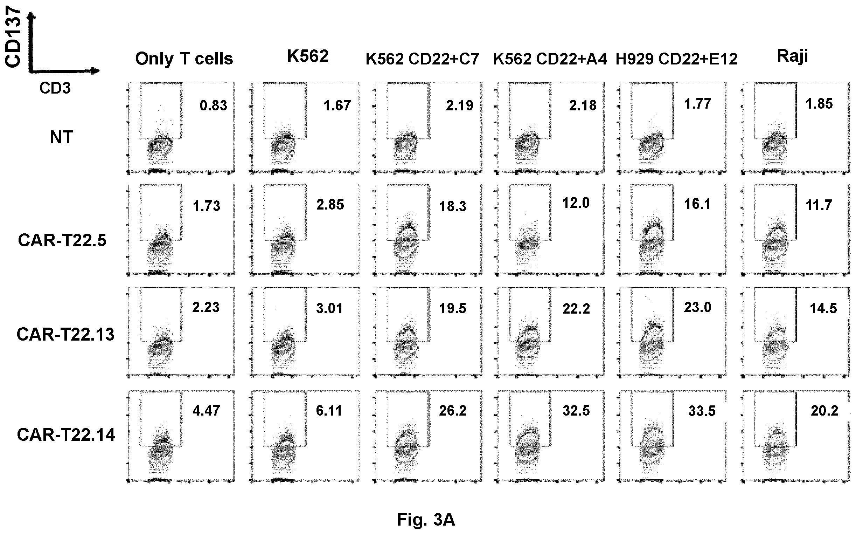

Figure 3A shows the expression level of CD137 on the surface of T cell membranes.

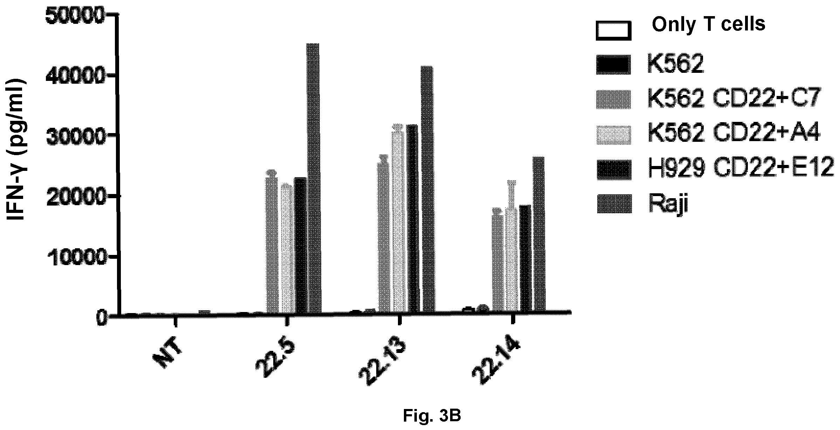

Figure 3B shows the secretion level of IFNγ in the culture supernatant. Specifically, 1 × 10⁵ CART-CD22s cells cultured to Day 6 were taken and cultured respectively with CD22-positive K562-CD22+C7, K562-CD22+A4, H929-CD22+E12, Raji line tumor cells, and CD22-negative K562 line tumor cells or tumor-free cells at a ratio of 1:1 in 200 µl GT-551 medium for 18 hours, and then the expression level of CD137 on the surface of the T cell membrane and the secretion level of IFN-γ in the culture supernatant were measured, respectively.

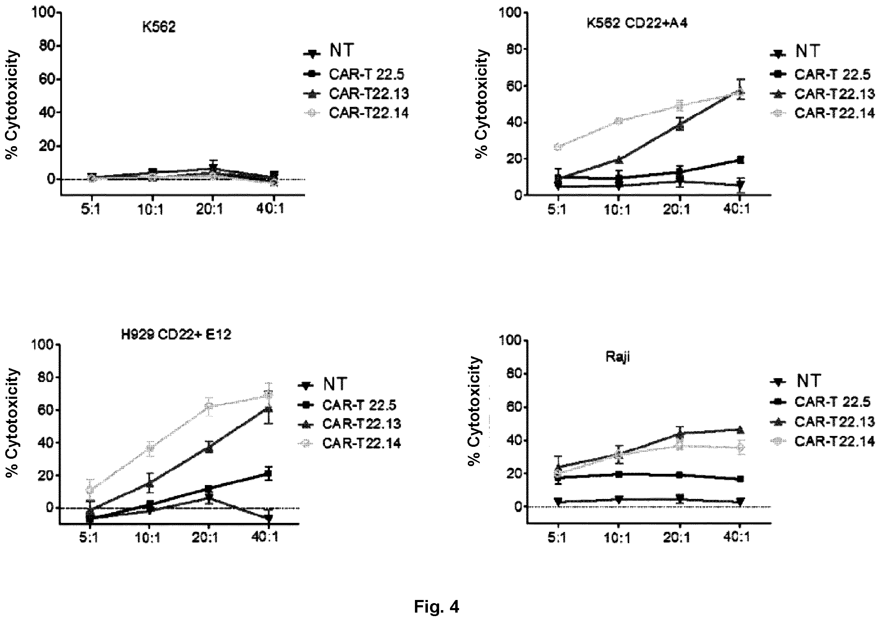

Figure 4 shows the assay of CART-CD22s-induced tumor cytotoxicity. Specifically, 1 × 10⁴ CD22-negative tumor cells (line K562) or CD22-positive tumor cells (line K562-CD22+A4, H929-CD22+E12, and Raji) were taken and respectively co-cultured with the corresponding CART cells at the ratios shown in the figure in 100 µl GT-551 medium for 8 hours, 50 µl of the medium supernatant was taken, 50 µl of the chromogenic substrate mixture was added, and a 30-minute coupled enzyme reaction was performed to detect the amount of lactate dehydrogenase (LDH) released during lysis of the tumor cells after being recognized and killed by the CAR-T cells, wherein the amount of the red product produced is proportional to the number of lysed cells. This figure shows the percentage of CART-CD22s-induced tumor cytotoxicity.

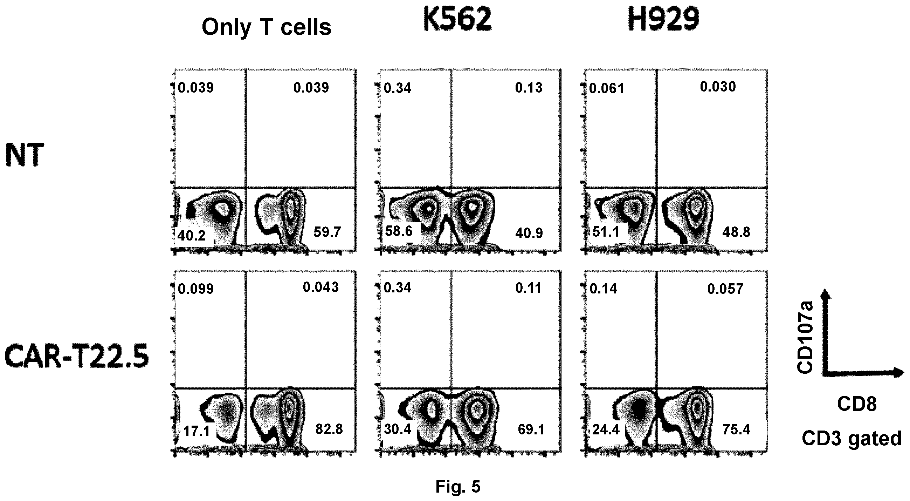

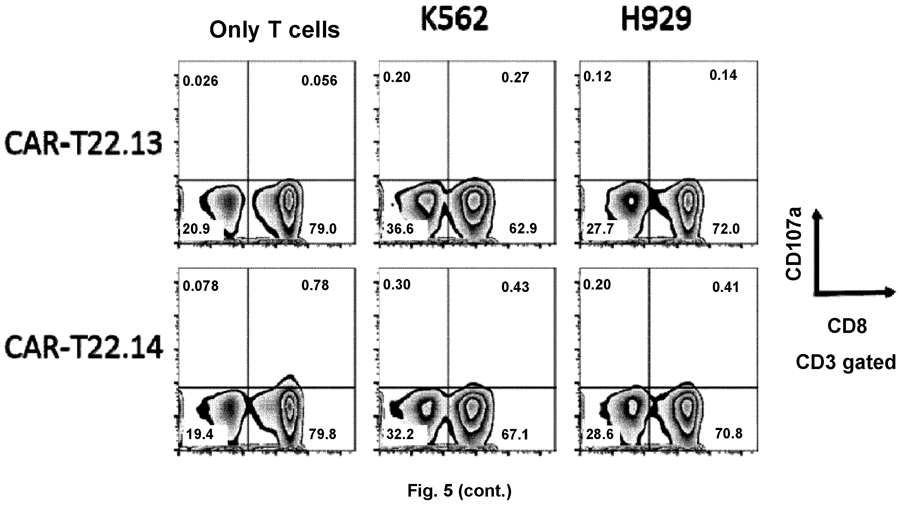

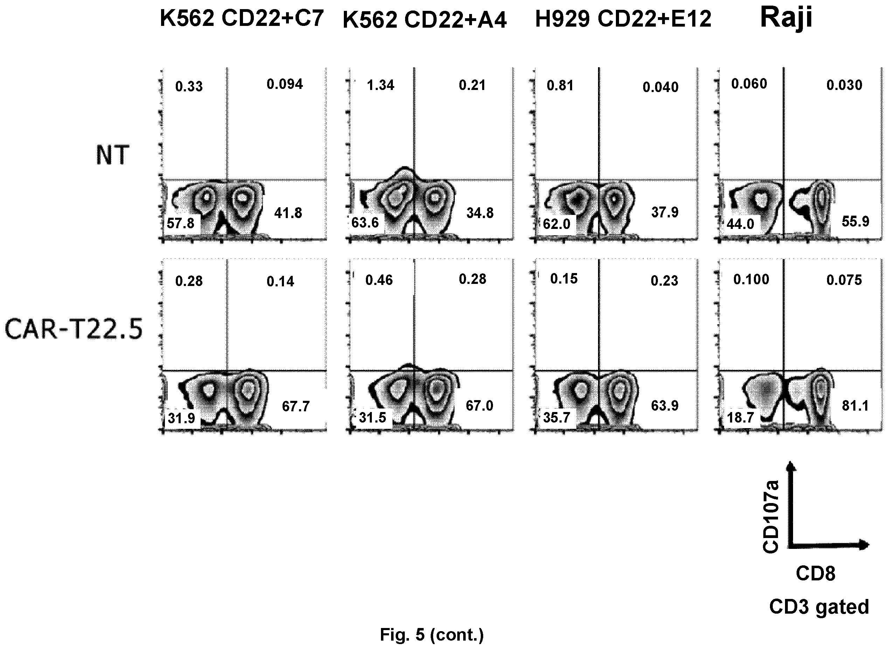

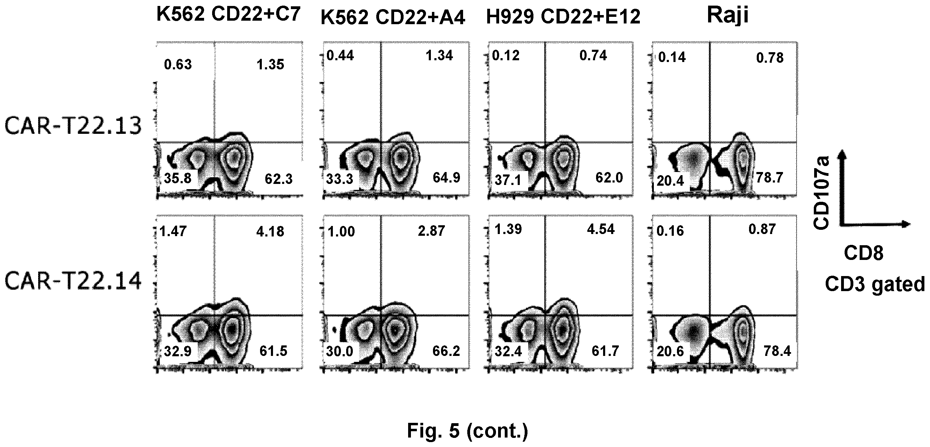

Figure 5 shows the assay of CD107a release levels during the late apoptosis process of CART-CD22s-induced tumor cells. Specifically, 2 × 10⁵ CD22-negative tumor cells (line K562) or CD22-positive tumor cells (lines K562-CD22+A4, H929-CD22+E12, and Raji) and 1 × 10⁵ CAR-T cells were taken and cultured in 200 µl GT-551 medium for 5 hours at a ratio of 1:1 by volume, and the change in the expression level of the membrane protein CD107a associated with T cell degranulation after CAR-T cells were activated by CD22-positive tumor cells was detected.

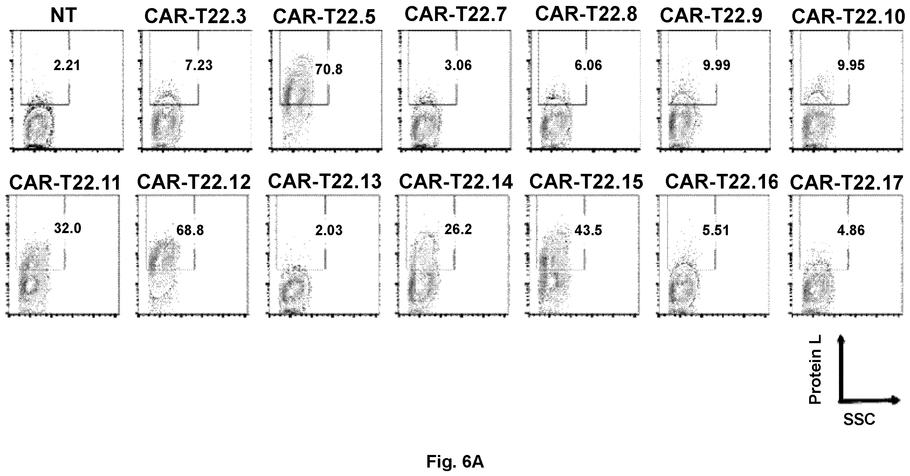

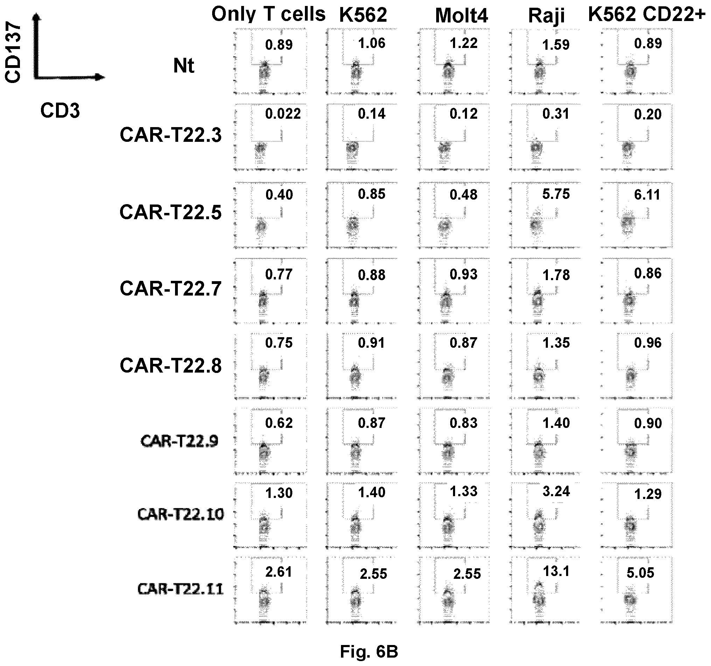

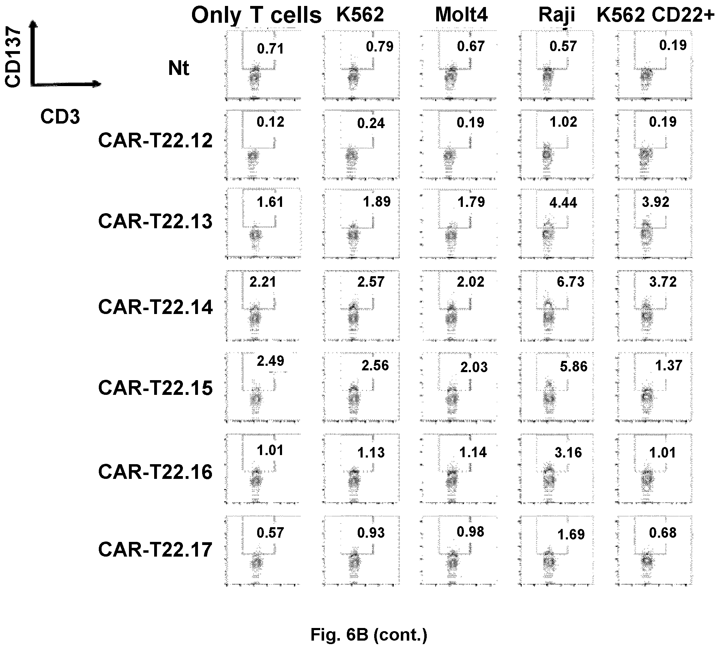

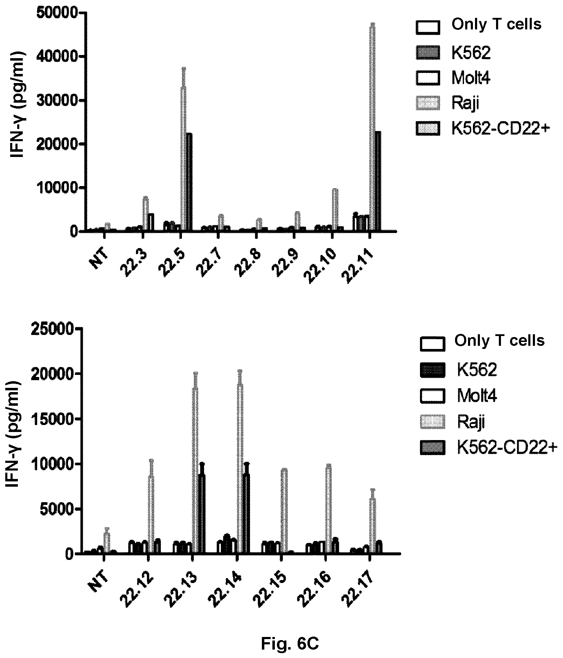

Figures 6A, 6B and 6C are the results of screening of CART-CD22s comparative examples. Figure 6A shows the measurement of the transfection efficiency of T cells engineered by a CD22-targeted chimeric antigen receptor. The expression level of CAR gene-encoded proteins on the surface of T cell membranes in CART-CD22s cells cultured up to Day 6 was detected by protein L staining. 1 × 10⁵ CART-CD22s cells cultured up to Day 6 were taken, mixed respectively with CD22-positive K562-CD22+ tumor cells, naturally CD22-expressing Raji line tumor cells, and CD22-negative K562 tumor cells or tumor-free cells, and cultured in 200 µl GT-551 medium at a ratio of 1:1 for 18 hours, and the expression level of CD137 on the surface of T cell membranes (Figure 6B) and the secretion level of IFNγ in the culture supernatant (Figure 6C) were detected, respectively.

### Specific embodiments

Through extensive and in-depth research and repeated screening, the inventors discovered for the first time a chimeric antigen receptor that can effectively target CD22. The extracellular antigen binding domain of the chimeric antigen receptor includes the antibody heavy chain variable region shown in SEQ ID NO.: 1 and the antibody light chain variable region shown in SEQ ID NO.: 2; or the antibody heavy chain variable region shown in SEQ ID NO.: 3 and the antibody light chain variable region shown in SEQ ID NO.: 4. In the present invention, CAR-T cells containing the chimeric antigen receptor were also prepared. The experimental results show that the chimeric antigen receptor and the CAR-T cells thereof provided by the present invention exhibited markedly high killing ability against tumor cells, the expression level of CD107a released increased, and the specificity was good, thereby effectively inducing apoptosis of CD22-positive tumor cells, hence the present invention.

Specifically, the antibodies screened in the present invention included CAR-T22.3, CAR-T22.5, CAR-T22.7, CAR-T22.8, CAR-T22.9, CAR-T22.10, CAR-T22.11, CAR-T22.12, CAR-T22.13, CAR-T22.14, CAR-T22.15, CAR-T22.16, and CAR-T22.17. Among them, CAR-T22.3 and CAR-T22.5 are published CAR-T sequences and were used as positive controls for screening. The results of the screening showed that the CAR-T cells obtained by CAR-T22.13, CAR-T22.14 and CAR-T22.5 had similar in vitro functions, and further experimental studies were carried out on these three CAR-T cells to detect and analyze the expression level, in vitro activation ability and tumor cell killing efficacy of these chimeric antigen receptors in primary T cells. The studies show that the chimeric antigen receptor of the present invention targeted CD22-positive cells and could be used for treating CD22-positive B-cell leukemias, including some CD19-negative and CD22-positive patients in which acute B-cell leukemia has recurred after anti-CD19 CAR-T treatments.

Specifically, the present invention determined the correlation between the expression time and expression intensity of different CAR structures on the surface of the cell membrane after virus infection, and then identified the differences in the ease of expression of proteins of different CAR structures. This finding suggested that different CAR structures under the same infection conditions differed in the expression level of the CAR protein on the membrane surface and the persistence of CART activity in vivo. After extensive screening, the CAR structures (CAR-T22.13 and CAR-T22.14) of the present invention were obtained. The results show that the proteins encoded by the CAR structures in the present invention could be sufficiently expressed and localized on the membrane. In addition, they had a strong ability to induce late apoptosis of tumor cells, a higher CD137 activation level and CD107a release level, and a better killing effect.

The present invention also improved the preparation process of T cells modified by CD22-targeted CAR structures, mainly by selecting GT-551 serum-free medium supplemented with 1% human albumin to culture lymphocytes in vitro.

### Terms

For a better understanding of the present disclosure, some terms are firstly defined. As used in this application, unless expressly stated otherwise herein, each of the following terms shall have the meaning given below. Additional definitions are also given throughout the application.

The term "approximately" may refer to a value or composition within an acceptable error range of a particular value or composition as determined by those ordinarily skilled in the art, which will depend in part on how the value or composition is measured or determined.

The term "administration" refers to the physical introduction of a product of the invention into a subject using any of a variety of methods and delivery systems known to those skilled in the art, including intravenously, intramuscularly, subcutaneously, intraperitoneally, spinally or other routes of parenteral administration, such as by injection or infusion.

The term "antibody" (Ab) shall include, but is not limited to, an immunoglobulin that specifically binds an antigen and comprises at least two heavy (H) chains and two light (L) chains interconnected by disulfide bonds, or its antigen-binding part. Each H chain comprises a heavy chain variable region (abbreviated as VH herein) and a heavy chain constant region. The heavy chain constant region contains three constant domains, CH1, CH2 and CH3. Each light chain comprises a light chain variable region (abbreviated as VL herein) and a light chain constant region. The light chain constant region contains one constant domain, CL. The VH and VL regions can be further subdivided into hypervariable regions called complementarity determining regions (CDRs), which contain dispersed, more conserved regions called framework regions (FRs). Each VH and VL contains three CDRs and four FRs, arranged from amino-terminus to carboxy-terminus in the following order: FR1, CDR1, FR2, CDR2, FR3, CDR3, FR4. The variable regions of the heavy chain and light chain contain binding domains that interact with an antigen.

### CD22

As a type I transmembrane protein and an important member of the sialic acid-binding immunoglobulin-like lectin family, CD22 has cell surface adhesion molecules that regulate B cell activation, helps control the sensitivity of B cells to antigen responses, and is prevalent in normal B cells and B cell malignancies. It is also known as BL-CAM, B3, Leu-14, Lyb-8, etc. The human CD22 gene is located on the long arm of chromosome 19 (19ql3.1), with at least 15 exons, of which exons 4-10 encode the single-chain Ig domain, and exons 11-15 encode the transmembrane domain and the intracellular domain. CD22 has two subtypes, namely, CD22α and CD22β, with 5 and 7 IgG domains in the extracellular domain, respectively.

CD22 is initially expressed in the cytoplasm of late pre-B cells, and then transferred to the cell surface. It is expressed in small amounts in pre-B cells and immature B cells and is highly expressed in differentiated mature immunoglobulin (Ig) M+ and IgD+ B cells. About 60-80% of B-cell malignancies express CD22, which is relatively specifically expressed on the surface of B cells and is still expressed at a relatively high level when CD19-targeted CART therapy fails and the malignancies relapse. A large number of experiments have proved that monoclonal antibodies targeting CD22 have significant efficacy for the treatment of leukemia. Therefore, it has become the target for regulating B-cell immunity and immunotherapy of B-cell malignancies.

Although anti-CD19 CAR-T has outstanding efficacy, it is ineffective for some leukemia patients who have missing CD19 antigen expression in B cells; some patients have decreased or even lost expression of CD19 antigen after anti-CD19 CAR-T treatment, resulting in unsatisfied treatment outcome and easy relapse while still having persistent expression of CD22. CD22 is expressed in most patients with B-cell acute lymphoblastic leukemia, including some CD19-negative patients after anti-CD19 CAR-T therapy. Fry et al. conducted a clinical trial of CAR-T therapy targeting CD22 antigens, where 73% (11/15) of the patients achieved complete remission after receiving ≥ 1 × 10⁶ anti-CD22 CAR-T cell therapy. Ramakrishna et al. believed that, by increasing the CD22 antigen expression in tumor cells, the efficacy of anti-CD22 CAR-T in the treatment of B-cell leukemias/lymphomas with low CD22 expression could be improved, but its further efficacy needs to be verified in animal experiments and clinical trials.

Recently, immunotherapy, especially adoptive T-cell therapy, has shown strong efficacy and promise in clinical trials for the treatment of hematological malignancies. T cells can be genetically modified to express a chimeric antigen receptor (CAR), which includes an antigen recognition moiety and a T cell activation domain. CARs use the antigen-binding properties of monoclonal antibodies to redirect T cell specificity and reactivity to aim at the targets in an MHC-unrestricted manner. This MHC-unrestricted antigen recognition enables CAR-expressing T cells to recognize antigens without antigen processing, thus avoiding a major mechanism of tumor escape. In addition, CARs do not dimerize with the α chain and β chain of endogenous TCR.

### Chimeric antigen receptor (CAR)

The chimeric antigen receptor (CAR) of the present invention includes an extracellular domain, a transmembrane domain, and an intracellular domain. The extracellular domain includes a target-specific binding element (also known as antigen-binding domain). The intracellular domain includes a costimulatory signaling region and a ζ chain portion. The costimulatory signaling region refers to a portion of an intracellular domain that includes a costimulatory molecule. Rather than antigen receptors or their ligands, costimulatory molecules are cell surface molecules that are required for an efficient lymphocyte response to an antigen.

A linker can be incorporated between the extracellular domain and the transmembrane domain of the CAR, or between the cytoplasmic domain and the transmembrane domain of the CAR. As used herein, the term "linker" generally refers to any oligopeptide or polypeptide that functions to link the transmembrane domain to the extracellular or cytoplasmic domain of a polypeptide chain. The linker may comprise 0-300 amino acids, preferably 2 to 100 amino acids, and most preferably 3 to 50 amino acids.

In a preferred embodiment of the present invention, the extracellular domain of the CAR provided by the present invention includes an antigen binding domain targeting CD22. The CAR of the present invention, when expressed in T cells, is capable of antigen recognition based on antigen binding specificity. When it binds to its cognate antigen, it affects tumor cells, causing the tumor cells to not grow, to die, or to be otherwise affected, and causing a reduction or elimination of the patient's tumor load. The antigen binding domain is preferably fused to one or more intracellular domains of the costimulatory molecule and the ζ chain. Preferably, the antigen binding domain is fused to the 4-1BB signaling domain, and to the intracellular domain of a combination with the CD3 ζ signaling domain.

As used herein, an "antigen binding domain" and a "single chain antibody fragment" both refer to a Fab fragment, a Fab' fragment, a F(ab')₂ fragment, or a single Fv fragment, which has antigen binding activity. The Fv antibody contains an antibody heavy chain variable region, a light chain variable region, but no constant regions, and is the smallest antibody fragment with all antigen-binding sites. Typically, the Fv antibody also contains a polypeptide linker between the VH and VL domains and is capable of forming a structure required for antigen binding. The antigen binding domain is usually an scFv (single-chain variable fragment). The size of the scFv is generally 1/6 of that of a complete antibody. The single chain antibody is preferably one amino acid chain sequence encoded by one nucleotide chain. As a preferred mode of the present invention, the scFv comprises an antibody that specifically recognizes CD22.

For the hinge region and the transmembrane region (transmembrane domain), the CAR can be designed to include a transmembrane domain fused to the extracellular domain of the CAR. In one embodiment, the transmembrane domain naturally associated with one of the domains in the CAR is used. In some examples, the transmembrane domain may be selected, or modified by amino acid substitutions to avoid binding such domains to transmembrane domains of the same or different surface membrane proteins, thereby minimizing the interaction with other members of the receptor complex.

The intracellular domains in the CAR of the present invention include the signaling domain of 4-1BB and the signaling domain of CD3 ζ.

Preferably, the structure of the CAR of the present invention comprises a leader sequence, an antigen recognition sequence (antigen binding domain), a connecting region, a transmembrane region, a costimulater signal region and a CD3 zeta signaling region (ζ chain portion), which are connected in the order below:
[CD8_LS]-[VL-Linker-VH]-[hinge-CD8TM]-[4-1BB]-[CD3 zeta]

Specifically, the following sequences are selected in the present invention:
(1) The leader sequence is that of the CD8 antigen:
   MALPVTALLLPLALLLHAARP (SEQ ID NO.: 9)
(2) CAR22.5 antigen recognition sequence: VH 
EVQLVESGGGLVQPGGSLRLSCAASGFAFSIYDMSWVRQVPGKGLEWVSYISSGGGTTYYPDTVKG
RFTISRDNSRNTLDLQMNSLRVEDTAVYYCARHSGYGSSYGVLFAYWGQGTLVTVSS (SEQ ID NO: 5)
 VL 
DIQMTQSPSSLSASVGDRVTITCRASQDISNYLNWLQQKPGKAPKLLIYYTSILHSGVPSRFSGSG
SGTEFTLTISSLQPEDFATYYCQQGNTLPWTFGQGTKLEIK (SEQ ID NO: 6)
(3) CAR22.3 antigen recognition sequence: VH 
LQQSGPGLVKPSQTLSLTCAISGDSVSSNSAAWNWIRQSPSRGLEWLGRTYYRSKWYNDYAVSVKS
RITINPDTSKNQFSLQLNSVTPEDTAVYYCAREVTGDLEDAFDIWGQGTMVTVS (SEQ ID NO: 7)
 VL 
IQMTQSPSSLSASVGDRVTITCRASQTIWSYLNWYQQRPGKAPNLLIYAASSLQSGVPSRFSGRGSG
TDFTLTISSLQAEDFATYYCQQSYSIPQTFGQGTKLEI (SEQ ID NO: 8)
(4) CAR22.13 antigen recognition sequence: VH 
EVQLVQSGAEVKKPGESLKISCKGSGYNFTSYWIGWVRQMPGKGLEWMGIIYPGDSDTRYSPSFQG
QVTISADKSISTAYLQWSSLKASDTAMYYCATPTYYFGSVAFDIWGQGTMVTVSS (SEQ ID NO: 1)
 VL 
EIVLTQSPDFQSVTPKEKVTITCRASQSIGSSLHWYQQKPDQSPKLLIKYASQSFSGVPSRFSGSG
SGTDFTLTINSLEAEDAAAYYCHQSSSLPYTFGQGTKLEIK (SEQ ID NO: 2)
(5) CAR22.14 antigen recognition sequence: VH 
QVQLVQSGAEVKKPGSSVKVSCKPSGDTFSNYAISWVRQAPGQGLEWMGRIIPILGMAIYAPKFQG
RVTITADKSTNTAFMDLTSLYFEDTAVYYCARAPTYWGSKDYFDYWGQGTLVTVSS (SEQ ID NO: 3)
 VL 
AIQLTQSPSSLSASVGDRVTITCRASQDISSGLAWYQQKPGTAPKLLIYDASSLESGVPSRFSGSG
SGTDFTLTISSLQPDDFATYYCQQFNSFPYTFGQGTKLEIK (SEQ ID NO: 4)
 In the above antigen recognition sequences, the connecting sequence between VH and VL is as shown in SEQ ID NO.: 16, and the sequence is: GGGGSGGGGSGGGGS.
(6) The sequence of the hinge region and the connecting region:
   FVPVFLPAKPTTTPAPRPPTPAPTIASQPLSLRPEACRPAAGGAVHTRGLDFACD (SEQ I D NO: 10)
(7) The sequence of the transmembrane region of CD8 (CD8TM) for the transmembrane region:
   IYIWAPLAGTCGVLLLSLVITLYC (SEQ ID NO.: 11)
(8) The costimulater signal region comes from the sequence of the intracellular signaling sequence motif of 4-1BB:
   KRGRKKLLYIFKQPFMRPVQTTQEEDGCSCRFPEEEEGGCEL (SEQ ID NO.: 12)
(9) The signaling region of CD3 zeta comes from the sequence of the immunoreceptor tyrosine-based activation motif (ITAM) of CD3 zeta in the TCR complex:

RVKFSRSADAPAYQQGQNQLYNELNLGRREEYDVLDKRRGRDPEMGGKPQRRKNPQEGLYNELQKD
KMAEAYSEIGMKGERRRGKGHDGLYQGLSTATKDTYDALHMQALPPR (SEQ ID NO: 13)

Preferably, the CAR of the present invention is CAR-T 22.13 or CAR-T 22.14, and the V_{L} and V_{H} in the CAR structure come from U.S. patent No. US9499632B2, wherein 16F7 is 22.13 and 4G6 is 22.14.

The amino acid sequence of CAR-T22.13 is as follows:

MALPVTALLLPLALLLHAARPEVQLVQSGAEVKKPGESLKISCKGSGYNFTSYWIGWVRQMPGKGLE
WMGIIYPGDSDTRYSPSFQGQVTISADKSISTAYLQWSSLKASDTAMYYCATPTYYFGSVAFDIWGQGTMV
TVSSGGGGSGGGGSGGGGSEIVLTQSPDFQSVTPKEKVTITCRASQSIGSSLHWYQQKPDQSPKLLIKYAS
QSFSGVPSRFSGSGSGTDFTLTINSLEAEDAAAYYCHQSSSLPYTFGQGTKLEIKFVPVFLPAKPTTTPAP
RPPTPAPTIASQPLSLRPEACRPAAGGAVHTRGLDFACDIYIWAPLAGTCGVLLLSLVITLYCKRGRKKLL
YIFKQPFMRPVQTTQEEDGCSCRFPEEEEGGCELRVKFSRSADAPAYQQGQNQLYNELNLGRREEYDVLDK
RRGRDPEMGGKPRRKNPQEGLYNELQKDKMAEAYSEIGMKGERRRGKGHDGLYQGLSTATKDTYDALHMQA
LPPR (SEQ ID NO: 14)

The amino acid sequence of CAR-T22.14 is as follows:

MALPVTALLLPLALLLHAARPQVQLVQSGAEVKKPGSSVKVSCKPSGDTFSNYAISWVRQAPGQGLE
WMGRIIPILGMAIYAPKFQGRVTITADKSTNTAFMDLTSLYFEDTAVYYCARAPTYWGSKDYFDYWGQGTL
VTVSSGGGGSGGGGSGGGGSAIQLTQSPSSLSASVGDRVTITCRASQDISSGLAWYQQKPGTAPKLLIYDA
SSLESGVPSRFSGSGSGTDFTLTISSLQPDDFATYYCQQFNSFPYTFGQGTKLEIKFVPVFLPAKPTTTPA
PRPPTPAPTIASQPLSLRPEACRPAAGGAVHTRGLDFACDIYIWAPLAGTCGVLLLSLVITLYCKRGRKKL
LYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQ
ALPPR (SEQ ID NO: 15)

### Chimeric antigen receptor T cells (CAR-T cells)

As used herein, terms "CAR-T cell," "CAR-T," "CART" and "CAR-T cell of the present invention" all refer to the CAR-T cell described in the fourth aspect of the present invention.

The present invention relates to the construction of a CD22-targeted chimeric antigen receptor structure, a method for preparing a CD22-targeted chimeric antigen receptor engineered T cell, and activity assay thereof.

In the present invention, based on the sequence of CD22, various types of chimeric antigen receptor structures targeting CD22 antigens were constructed, and the expression level, in vitro activation ability and tumor cell killing efficacy of these chimeric antigen receptors in primary T cells were measured and analyzed. The present invention found the differences in the ability of different types of CAR-modified T cells constructed on the basis of CAR22.13 and 22.14 to kill in vitro and in vivo to remove malignant tumors carrying CD22 antigens, which provides a new effective method and a formulation for clinical application of CAR-T in the treatment of CD22-positive leukemias and lymphomas.

### Vector

A nucleic acid sequence encoding a desired molecule can be obtained using recombinant methods known in the art, for example, by screening libraries from cells expressing the gene, by obtaining the gene from a vector known to include the gene, or by using standard technology to isolate the gene directly from cells and tissues that contain it. Alternatively, the gene of interest can be produced synthetically.

The present invention also provides a vector into which the expression kit of the present invention is inserted. Vectors derived from retroviruses such as lentiviruses are suitable tools for long-term gene transfer because they allow long-term, stable integration of the transgene and the proliferation thereof in its daughter cells. Lentiviral vectors have advantages over vectors derived from oncogenic retroviruses such as murine leukemia viruses because they can transduce non-proliferating cells such as hepatocytes. They also have the advantage of low immunogenicity.

In short, an expression kit or nucleic acid sequence of the present invention is typically operably linked to a promoter and incorporated into an expression vector. The vector is suitable for replicating and integrating eukaryotic cells. Typical cloning vectors include transcriptional and translational terminators, initial sequences and promoters that can be used to regulate the expression of the desired nucleic acid sequence.

The expression constructs of the present invention can also be used in nucleic acid immunization and gene therapy using standard gene delivery solutions. Methods for gene delivery are known in the art; see, e.g., U.S. Patent Nos. 5,399,346, 5,580,859, 5,589,466, which are incorporated herein in their entirety by reference. In another embodiment, the present invention provides gene therapy vectors.

The nucleic acid can be cloned into many types of vectors. For example, the nucleic acid can be cloned into vectors including, but not limited to, plasmids, phagemids, phage derivatives, animal viruses, and cosmids. Particular vectors of interest include expression vectors, replication vectors, probe generation vectors, and sequencing vectors.

Further, expression vectors can be provided to cells in the form of viral vectors. Viral vector techniques are well known in the art and are described, for example, by Sambrook et al. (2001, Molecular Cloning: A Laboratory Manual, Cold Spring Harbor Laboratory, New York) and in other manuals of virology and molecular biology. Viruses that can be used as vectors include, but are not limited to, retroviruses, adenoviruses, adeno-associated viruses, herpesviruses, and lentiviruses. Typically, suitable vectors contain an origin of replication functional in at least one organism, a promoter sequence, convenient restriction enzyme sites, and one or more selectable markers (e.g., WO01/96584; WO01/29058; and U.S. Patent No. 6,326,193).

A number of virus-based systems have been developed for transfer of genes into mammalian cells. For example, retroviruses provide a convenient platform for gene delivery systems. The selected gene can be inserted into a vector and packaged into retroviral particles using techniques known in the art. The recombinant virus can then be isolated and delivered to subject cells in vivo or ex vivo. Many retroviral systems are known in the art. In some embodiments, adenoviral vectors are used. Many adenoviral vectors are known in the art. In one embodiment, lentiviral vectors are used.

Additional promoter elements, such as enhancers, can regulate the frequency at the start of transcription. Typically, these are located in a region of 30-110 bp upstream of the start site, although it has recently been shown that many promoters also contain functional elements downstream of the start site. The spacing between promoter elements is often flexible so that the promoter function is maintained when elements are inverted or moved relative to one another. In the thymidine kinase (tk) promoter, the spacing between promoter elements can be increased by 50 bp before activity begins to decline. Depending on the promoter, individual elements demonstrate cooperative or independent action to initiate transcription.

An example of a suitable promoter is the immediate early cytomegalovirus (CMV) promoter sequence. The promoter sequence is a strong constitutive promoter sequence capable of driving high-level expression of any polynucleotide sequence operably linked to it. Another example of a suitable promoter is elongation growth factor-lα (EF-1α). However, other constitutive promoter sequences can also be used, which include but are not limited to the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate-early promoter, Rous sarcoma virus promoter, and human gene promoters, for example but not limited to the actin promoter, myosin promoter, heme promoter and creatine kinase promoter. Further, the present invention should not be limited to the use of constitutive promoters. Inducible promoters are also contemplated as part of the present invention. The use of an inducible promoter provides a molecular switch that can turn on the expression of a polynucleotide sequence operably linked to the inducible promoter when such expression is desired or turn off the expression when the expression is not desired. Examples of inducible promoters include, but are not limited to, the metallothionein promoter, glucocorticoid promoter, progesterone promoter, and tetracycline promoter.

To evaluate the expression of a CAR polypeptide or portion thereof, the expression vector introduced into cells may also contain either or both of a selectable marker gene and a reporter gene to facilitate the search for identification and selection of expressing cells in the transfected or infected cell population through the viral vector. In other aspects, the selectable marker can be carried on a single section of DNA and used in co-transfection procedures. Both the selectable marker and the reporter gene can be flanked by appropriate regulatory sequences to enable expression in the host cell. Useful selectable markers include, for example, antibiotic resistance genes such as neo and the like.

A reporter gene is used to identify potentially transfected cells and to evaluate the functionality of regulatory sequences. Typically, a reporter gene is a gene that is not present in or expressed by the recipient organism or tissue and that encodes a polypeptide whose expression is clearly indicated by some readily detectable properties such as enzymatic activity. After the DNA has been introduced into the recipient cell, the expression of the reporter gene is measured at an appropriate time. Suitable reporter genes may include genes encoding luciferase, β-galactosidase, chloramphenicol acetyltransferase, secreted alkaline phosphatase, or green fluorescent protein (e.g., Ui-Tei et al., 2000 FEBS Letters 479: 79-82). Suitable expression systems are well known and can be prepared using known techniques or obtained commercially. Typically, constructs with a minimum of 5 flanking regions showing the highest levels of reporter gene expression are identified as promoters. Such promoter regions can be linked to a reporter gene and used to evaluate the ability of an agent to regulate promoter-driven transcription.

Methods for introducing and expressing genes into cells are known in the art. In terms of the content of an expression vector, the vector can be easily introduced by any method known in the art into a host cell, e.g., mammalian, bacterial, yeast or insect cells. For example, an expression vector can be transferred into a host cell by physical, chemical or biological means.

Physical methods for introducing polynucleotides into host cells include calcium phosphate precipitation, lipofection, particle bombardment, microinjection, electroporation, and the like. Methods for producing cells comprising a vector and/or exogenous nucleic acids are well known in the art. See, e.g., Sambrook et al. (2001, Molecular Cloning: A Laboratory Manual, Cold Spring Harbor Laboratory, New York). The preferred method for introducing polynucleotides into host cells is calcium phosphate transfection.

Biological methods for introducing polynucleotides of interest into host cells include the use of DNA and RNA vectors. Viral vectors, especially retroviral vectors, have become the most widely used method of inserting genes into mammalian, e.g., human cells. Other viral vectors can be derived from lentiviruses, poxviruses, herpes simplex virus I, adenoviruses and adeno-associated viruses, etc. See, e.g., US Patent Nos. 5,350,674 and 5,585,362.

Chemical means of introducing polynucleotides into host cells include colloidal dispersion systems, such as macromolecular complexes, nanocapsules, microspheres, beads; and lipid-based systems, including oil-in-water emulsions, micelles, mixed micelles, and lipid plastids. Exemplary colloidal systems for use as in vitro and in vivo delivery vehicles are liposomes (e.g., artificial membrane vesicles).

Where non-viral delivery systems are used, exemplary delivery vehicles are liposomes. The use of lipid formulations is contemplated for introducing a nucleic acid into the host cell (in vitro, ex vivo, or in vivo). In another aspect, the nucleic acid can be associated with lipids. The nucleic acid associated with lipids can be encapsulated into the aqueous interior of liposomes, interspersed in the lipid bilayer of liposomes, attached to liposomes via a linker molecule associated with both the liposomes and oligonucleotides, entrapped in liposomes, complexed with liposomes, dispersed in lipid-containing solutions, mixed with lipids, associated with lipids, contained in lipids as a suspension, contained in micelles or complexed with micelles, or otherwise associated with lipids. The lipids, lipids/DNA or lipids/expression vector associated with the composition are not limited to any particular structure in a solution. For example, they may exist in bilayer structures, as micelles or have a "collapsed" structure. They can also simply be dispersed in a solution, possibly forming aggregates that are not uniform in size or shape. Lipids are fatty substances which can be naturally occurring or synthetic. For example, lipids include lipid droplets, which occur naturally in the cytoplasm as well as in such compounds comprising long chain aliphatic hydrocarbons and their derivatives such as fatty acids, alcohols, amines, amino alcohols and aldehydes.

In a preferred embodiment of the present invention, the vector is a lentiviral vector.

### Formulations

The present invention provides a CAR-T cell of the first aspect of the present invention, and a pharmaceutically acceptable carrier, diluent or excipient. In one implementation, the formulation is a liquid formulation. Preferably, the formulation is an injection. Preferably, the concentration of the CAR-T cells in the formulation is 1 × 10³-1 × 10⁸ cells/ml, more preferably 1 × 10⁴-1 × 10⁷ cells/ml.

In one embodiment, the formulation may include a buffer such as neutral buffered saline, sulfate buffered saline, etc.; carbohydrates such as glucose, mannose, sucrose or dextran, mannitol; proteins; polypeptides or amino acids such as glycine; antioxidants; chelating agents such as EDTA or glutathione; adjuvants (e.g., aluminum hydroxide); and preservatives. The formulations of the present invention are preferably formulated for intravenous administration.

### Therapeutic use

The present invention includes therapeutic use of cells (e.g., T cells) transduced with lentiviral vectors (LVs) encoded with the expression kit of the present invention. The transduced T cells can target the tumor cell marker CD22, synergistically activate T cells, and cause T cell immune responses, thereby significantly improving their killing efficiency against tumor cells.

Accordingly, the present invention also provides a method of stimulating a T cell-mediated immune response to a target cell population or tissue in a mammal, comprising a step of administering the CAR-T cells of the present invention to the mammal.

In one embodiment, the present invention includes a cell therapy, wherein a patient's autologous T cells (or allogeneic donor) are isolated, activated and genetically engineered to produce CAR-T cells, which are then infused into the patient. In this way, the probability of graft-versus-host disease is extremely low, and the antigen is recognized by T cells in an MHC-unrestricted way. In addition, one CAR-T cell can treat all cancers that express this antigen. Unlike antibody therapies, CAR-T cells can replicate in vivo, resulting in long-lasting sustained tumor control.

In one embodiment, the CAR-T cells of the present invention can have robust in vivo proliferation of T cells for an extended period of time. Additionally, a CAR-mediated immune response can be part of an adoptive immunotherapy step in which CAR-modified T cells induce an immune response specific to the antigen binding domain in the CAR. For example, CD22-expressing tumor cells induce specific immune responses of anti-CD22 CAR-T cells.

Although the data of this disclosure specifically discloses lentiviral vectors comprising anti-CD22 scFv, hinge and transmembrane regions, and 4-1BB and CD3 ζ signaling domains, the present invention should be construed as including any variation in the number of each of the components of the construct.

Cancers that can be treated include tumors that are not vascularized or not substantially vascularized, as well as tumors that are vascularized. Cancers may include non-solid tumors (such as hematological tumors, e.g., leukemias and lymphomas) or may include solid tumors. Cancer types that are treated with the CARs of the present invention include but are not limited to carcinomas, blastomas, and sarcomas, and certain leukemic or lymphoid malignancies, benign and malignant tumors, and malignant masses such as sarcomas, carcinomas, and melanomas. Adult tumors/cancers and childhood tumors/cancers are also included.

Hematological cancers are cancers of the blood or bone marrow. Examples of hematological (or hematogenous) cancers include leukemias, including acute leukemias (such as acute lymphoblastic leukemia, acute myeloid leukemia, acute myelogenous leukemia, and myeloblastoid, promyelocytic, granulocytic, monocytic and erythroleukemia), chronic leukemias (such as chronic myeloid (granulocytic) leukemia, chronic myelogenous leukemia, and chronic lymphocytic leukemia), polycythemia vera, lymphoma, Hodgkin's disease, non-Hodgkin's lymphoma (painless and high-grade forms), multiple myeloma, Waldenstrom's macroglobulinemia, heavy chain disease, myelodysplastic syndrome, hairy cell leukemia, and myelodysplasia.

Solid tumors are abnormal masses of tissue that typically do not contain cysts or fluid regions. Solid tumors can be benign or malignant. Different types of solid tumors are named after the cell type that forms them (such as sarcomas, carcinomas, and lymphomas). Examples of solid tumors such as sarcomas and carcinomas include fibrosarcoma, myxosarcoma, liposarcoma, mesothelioma, lymphoid malignancies, pancreatic cancer, and ovarian cancer.

The CAR-modified T cells of the present invention can also be used as a type of vaccine for ex vivo immunization and/or in vivo therapy of mammals. Preferably, the mammal is a human.

For ex vivo immunization, at least one of the following occurs in vitro prior to administering the cells into a mammal: i) proliferating cells, ii) introducing the nucleic acid encoding the CAR into the cells, and/or iii) cryopreserving the cells.

Ex vivo procedures are well known in the art and are discussed more fully below. Briefly, cells are isolated from mammals (preferably humans) and genetically modified (i.e., transduced or transfected in vitro) with a vector expressing the CAR disclosed herein. The CAR-modified cells can be administered to mammalian recipients to provide therapeutic benefits. The mammalian recipient can be human, and the CAR-modified cells can be autologous to the recipient. Alternatively, the cells may be allogeneic, syngeneic or xenogeneic to the recipient.

In addition to using cell-based vaccines for ex vivo immunization, the present invention also provides compositions and methods for in vivo immunization to elicit an immune response against antigens in patients.

The present invention provides methods of treating tumors, comprising administering to a subject in need thereof a therapeutically effective amount of the CAR-modified T cells of the present invention.

The CAR-modified T cells of the invention can be administered alone or as a pharmaceutical composition in combination with diluents and/or with other components such as IL-2, IL-17 or other cytokines or cell populations. Briefly, the pharmaceutical compositions of the present invention may include the target cell populations as described herein, in combination with one or more pharmaceutically or physiologically acceptable carriers, diluents or excipients. Such compositions may include buffers such as neutral buffered saline, sulfate buffered saline, and the like; carbohydrates such as glucose, mannose, sucrose or dextran, mannitol; proteins; polypeptides or amino acids such as glycine; antioxidants; chelates such as EDTA or glutathione; adjuvants (e.g., aluminum hydroxide); and preservatives. The compositions of the present invention are preferably formulated for intravenous administration.

The pharmaceutical compositions of the present invention can be administered in a manner appropriate to the disease to be treated (or prevented). The amount and frequency of administration will be determined by factors such as the patient's condition, and the type and severity of the patient's disease, although appropriate dosages can be determined by clinical trials.

When referring to an "immunologically effective amount," "effective amount against tumors," "effective tumor-suppressing amount" or "therapeutic amount," the precise amounts of the compositions of the invention to be administered can be determined by physicians, taking into account the individual differences of patients (subjects) in terms of age, weight, tumor size, degree of infection or metastasis, and medical condition. It may generally be noted that the pharmaceutical compositions comprising the T cells described herein may be administered at doses ranging from 10⁴ to 10⁹ cells/kg body weight, preferably 10⁵ to 10⁶ cells/kg body weight (including all integer values within those ranges). The T cell compositions can also be administered multiple times at these doses. The cells can be administered using infusion techniques well known in immunotherapy (see, e.g., Rosenberg et al., NewEng. J. of Med. 319: 1676, 1988). The optimal dosage and treatment regimen for a particular patient can be easily determined by those skilled in the medical arts by monitoring the patient for signs of disease and adjusting treatment accordingly.

Administration of the compositions to subjects can be carried out in any convenient way, including by nebulization, injection, swallowing, infusion, implantation, or transplantation. The compositions described herein can be administered to patients subcutaneously, intradermally, intratumorally, intranodally, intraspinally, intramuscularly, by intravenous (i.v.) injection, or intraperitoneally. In one embodiment, the T cell compositions of the present invention are administered to patients by intradermal or subcutaneous injection. In another embodiment, the T cell compositions of the present invention are preferably administered by i.v. injection. The T cell compositions can be injected directly into tumors, lymph nodes or sites of infection.

In some embodiments of the present invention, the T cells activated and amplified to therapeutic levels using the methods described herein, or other methods known in the art, are combined with any number of relevant therapeutic modalities (e.g., before, concurrently or after) to patients, the therapeutic modalities including but not limited to treatment with agents such as antiviral therapy, cidofovir and interleukin-2, cytarabine (also known as ARA-C) or natalizumab therapy for MS patients or elfazizumab therapy for psoriasis patients or other treatments for PML patients. In a further embodiment, the T cells of the present invention may be used in combination with chemotherapy, radiation, immunosuppressive agents such as cyclosporine, azathioprine, methotrexate, mycophenolate mofetil and FK506, antibodies or other immunotherapeutic agents. In a further embodiment, the cell compositions of the present invention are administered (e.g., before, concurrently or after) to patients in combination with bone marrow transplantation, use of chemotherapeutic agents such as fludarabine, external beam radiation therapy (XRT), and cyclophosphamide. For example, in one embodiment, the subject may undergo standard treatment with high dose chemotherapy followed by peripheral blood stem cell transplantation. In some embodiments, after transplantation, the subject receives infusions of the amplified immune cells of the present invention. In an additional embodiment, the amplified cells are administered before or after surgery.

The dosages of the above treatments administered to patients will vary depending on the precise nature of the condition being treated and the recipients of the treatments. Dosage ratios for human administration can be implemented in accordance with the accepted practice in the art. Typically, for each treatment or each course of treatment, 1 × 10⁶ to 1 × 10¹⁰ modified T cells of the present invention (e.g., CART-CD22 cells) can be administered to a patient, e.g., by intravenous infusion.

### Main advantages of the present invention:

(a) The extracellular antigen binding domain of the chimeric antigen receptor of the present invention is a specific anti-CD22 scFv, and the CAR formed by the specific anti-CD22 scFv combined with a specific hinge region and intracellular domain showed great tumor cell killing ability, good specificity, low cytotoxicity to T cells, and low side effects.
(b) The chimeric antigen receptor provided by the present invention could realize stable expression and membrane localization of the CAR protein after the lentivirus carrying the CAR gene infected the T cells.
(c) The CAR-modified T cells of the present invention had a longer survival time in vivo and stronger anti-tumor efficacy; the antigen-binding domain used in the present invention was a humanized or human-derived antibody, which was less likely to produce species-specific immune rejection reactions.

The present invention is further described by referring to some embodiments. It should be understood that the embodiments are only used to illustrate the present invention but not to limit the scope of the present invention. In the following embodiments where no conditions are specified for the experimental method, conventional conditions are generally used, for example, the conditions described in Sambrook et al., Molecular Cloning: A Laboratory Manual (New York: Cold Spring Harbor Laboratory Press, 1989), or recommendations of the manufacturer. Unless otherwise specified, percentages and parts are calculated by weight.

### Example 1

### Construction of the lentiviral expression vector

Shanghai Boyi Biotechnology Co., Ltd. was commissioned to conduct full-length DNA synthesis and cloning to construct the encoding plasmid. The pWPT lentiviral vector was selected as the cloning vector, and the cloning sites were BamH I and Mlu I. The specific sequences were as described above.

### Example 2

### Preparation of CAR-T cells

(1) Venous blood was taken from a healthy human, and peripheral blood mononuclear cells (PBMCs) were isolated by density gradient centrifugation.
(2) On Day 0, the PBMCs were seeded into a cell culture flask pre-coated with CD3 monoclonal antibody (OKT3) of a final concentration of 5 µg/ml and Retronectin (purchased from TAKARA) of a final concentration of 5 µg/ml, in GT-551 cell culture medium containing 1% human blood albumin, supplemented with recombinant human interleukin 2 (IL-2) of a final concentration of 1,000 U/ml, and cultured in an incubator at 37°C with a saturated humidity of 5% CO₂.
(3) On Day 1, the supernatant of the cultured PBMCs was slowly pipetted and discarded, a new GT-551 cell culture medium containing 1% human albumin was added, recombinant human interleukin 2 (IL- 2) of the final concentration of 1,000 U/ml was added to the medium, and culturing was resumed in an incubator at 37°C with a saturated humidity of 5% CO₂.
(4) On Day 3, fresh medium was added, purified CAR-CD22s lentiviral fluid was concentrated, protamine sulfate (12 µg/ml) and IL-2 of a final concentration of 1,000 U/ml were added. It was placed in an incubator at 37°C with 5% CO₂ for infection for 12 hours, the culture solution was discarded, fresh medium was added, and culturing was resumed in an incubator at 37°C with 5% CO₂.
(5) From Day 6, CART-CD22s cells were taken for the required in vitro activity assays.

### Example 3

### Assay of the integration rate of the CAR gene in T cell genome and the expression level of the protein encoded thereby on the membrane surface

0.2 × 10⁶ of CART-CD22.5, CART-CD22.13 and CART-CD22.14 sample cells cultured to Day 6 in Example 2 (Figure 2) were taken, and, after staining the Fc segment of recombinant human CD22 protein, the expression level of CAR-CD22 protein on the surface of T cell membranes was analyzed by flow cytometry, with untransduced T cells (NT cells) used as control.

The results are shown in Figure 2. The CAR structures of the three CAR-T cells could be expressed in the correspondingly modified T cells and localized on the cell membrane surface and had a high expression rate. Among them, the expression rates of CART-CD22.5 and CART-CD22.14 were higher than that of CART-CD22.13.

### Example 4

### Assay of in vitro activation ability of CART-CD22s

The CART-CD22s cells (CART-CD22.5, CART-CD22.13 and CART-CD22.14) cultured in Example 2 were used for the detection of cell activation level indicator proteins CD137 and IFNγ. 1 × 10⁵ CART-CD22s cells cultured to Day 6 were taken, cultured respectively with CD22-positive K562-CD22+C7, K562-CD22+A4, H929-CD22+E12 and Raji tumor cells, and CD22-negative K562 tumor cells or tumor-free cells in 200 µl GT-551 medium at a ratio of 1:1 for 18 hours, and then the expression level of CD137 on the surface of T cell membrane was detected by the flow method, and the secretion level of IFNγ in the culture supernatant was detected by the ELISA method.

The results are shown in Figure 3A. The expression of CD137 could be detected on the surface of all the three CAR-T cells, and the expression of IFNγ could be detected in the culture supernatant. Among them, CAR-T22.13 and CAR-T22.14 had better CD137 activation levels than CAR-T22.5, but the IFNγ release level of CAR-T22.5 was higher than that of CAR-T22.13 and CAR-T22.14.

### Example 5

### Assay of the activity of the CART-CD22s to induce late apoptosis of tumor cells

The CART-CD22s cells to Day 14 in Example 2 were mixed respectively with 1 × 10⁴ CD22-negative cells (K562) or CD22-positive cells of the Raji line and autologous cells K562-CD22 overexpressing tumor cell lines at the ratio of 5:1, 10:1, 20:1, and 40:1, respectively, and co-cultured in a 100 µl GT-551 culture system for 8 hours, 50 µl supernatant of the medium was pipetted, chromogenic substrate mixture was added, and a 30-minute coupled enzyme reaction was performed to detect the amount of lactate dehydrogenase (LDH) released during lysis of the tumor cells after being recognized and killed by the CAR-T cells, wherein the amount of the red product produced was proportional to the number of lysed cells.

The results are shown in Figure 4. No T cells had the ability to kill the CD22-negative tumor cell line K562, and the three CAR-T cells could induce the apoptosis of CD22-positive tumor cells well. Among them, CAR-T22.13 and CAR-T22.14 could better induce late apoptosis of CD22-positive tumor cells than CAR-T22.5; CAR-T22.14 had slightly better ability to induce late apoptosis of CD22-positive tumor cells in CAR-T22.13.

### Example 6

### Assay of the expression level of membrane protein CD107a associated with T cell degranulation

2 × 10⁵ CD22-negative tumor cells (line K562) or CD22-positive tumor cells (lines K562-CD22+A4, H929-CD22+E12, and Raji) and 1 × 10⁵ CAR-T cells were taken and cultured in 200 µl GT-551 medium for 5 hours at a ratio of 1:1 by volume, and the change in the expression level of the membrane protein CD107a associated with T cell degranulation after CAR-T cells were activated by CD22-positive tumor cells was detected.

The results are shown in Figure 5. After the three CAR-T cells were activated by CD22-positive tumor cells (K562-CD22+C7, K562-CD22+A4, and H929-CD22+E12), the expression level of released CD107a increased, while the expression of CD107a in CAR-T cells did not increase significantly after co-culturing with Raji cells. Among them, the CD107a release of CAR-T22.14 was higher than that of CAR-T22.13 and CAR-T22.5, indicating a stronger killing effect.

### Comparative Example 1

The inventors tested a large number of candidate sequences during the screening of the chimeric antigen receptor structure of the present application. Candidate CART cells included CAR-T22.3, CAR-T22.5, CAR-T22.7, CAR-T22.8, CAR-T22.9, CAR-T22.10, CAR-T22.11, CAR-T22 .12, CAR-T22.15, CAR-T22.16, and CAR-T22.17, wherein the preparation method of CAR-T cells was the same as that of Example 2, and the assay methods were the same as those of Examples 3 and 4.

The antibody sequences included in candidate CAR-Ts came from published literature and patents. Among them, the sequences of the light chain and the heavy chain of the CD22 antibody (m971) used in CAR-T22.3 came from U.S. patent No. US8591889B2; the sequences of the light chain and the heavy chain of the CD22 antibody (KM196172) of CAR-T22.5 came from https://www.ncbi.nlm.nih.gov/nuccore/KM196172; the sequences of the light chain and the heavy chain of the CD22 antibody (VM1000) used in CAR-T22.7 came from U.S. patent No. US9856323B2; the sequences of the light chain and the heavy chain of the CD22 antibody (LL2) used in CAR-T22.8 came from literature (Pawlak Byczkowska, E. J. et al., Cancer Res. 49: 4568-4577(1989)); the sequences of the light chain and the heavy chain of the CD22 antibody (10F4) used in CAR-T22.9 came from U.S. patent No. US2014/0127197A1; the sequences of the light chain and the heavy chain of the CD22 antibody (19A3) used in CAR-T22.10, antibody (23C6) in 22.11, antibody (16F7) in 22.12, antibody (4G6) in 22.15, antibody (21F4) in 22.16 and antibody (21F4) in 22.17 came from U.S. patent No. US9499632B2.

The assay results of the CART cells are shown in Figures 6A, 6B and 6C. Through the assay of the positive rate (Figure 6A), CD137 activation ability in vitro (Figure 6B) and IFNγ release level in the supernatant after co-culturing with target cells (Figure 6C), it was found that the positive rates of CAR-T22.3, CAR-T22.7, CAR-T22.8, CAR-T22.9, CAR-T22.10, CAR-T22.16 and CAR-T22.17 were very low, and they were regarded as ineffective; it was found in the IFNγ test that IFNγ release was very high and CD137 expression was also high after CAR-T22.5, CAR-T22.11, CAR-T22.13 and CAR-T22.14 were co-cultured with CD22-positive tumor cells, indicating that these CAR-T cells were specifically activated. Therefore, 22.13 and 22.14 were selected for further studies.

All documents mentioned in the present invention are cited as references in this application as if each document is individually cited. In addition, it should be understood that, after reading the above teaching of the present invention, those skilled in the art can make various changes or modifications to the present invention, and these equivalent forms also fall within the scope defined by the appended claims of this application.

## Claims

1. A chimeric antigen receptor (CAR), **characterized in that** an antigen binding domain (i.e., scFv) of the chimeric antigen receptor comprises an antibody heavy chain variable region shown in SEQ ID NO.: 1 or 3 and an antibody light chain variable region shown in SEQ ID NO.: 2 or 4.

2. The CAR as described in claim 1, **characterized in that** the structure of the antigen binding domain is shown in formula I or II below:
V_{L}-V_{H} (I); V_{H}-V_{L} (II)
wherein V_{H} is the antigen heavy chain variable region; V_{L} is the antigen light chain variable region; "-" is a linking peptide or peptide bond; preferably, the structure of the antigen binding domain is as shown in formula I.

3. The CAR as described in claim 2, **characterized in that** the amino acid sequence of the V_{L} is shown in SEQ ID NO.: 1, and the amino acid sequence of the V_{H} is shown in SEQ ID NO.: 2.

4. The CAR as described in claim 2, **characterized in that** the amino acid sequence of the V_{L} is shown in SEQ ID NO.: 3, and the amino acid sequence of the V_{H} is shown in SEQ ID NO.: 4.

5. The CAR as described in claim 1, **characterized in that** the structure of the chimeric antigen receptor is shown in formula III below:
L-V_{L}-V_{H}-H-TM-C-CD3 ζ (III)
wherein,
L is an optional leader sequence, i.e., a signal peptide;
H is a hinge region;
TM is the transmembrane domain;
C is a costimulatory signal molecule;
CD3 ζ is a cytoplasmic signaling sequence derived from CD3ζ; and
V_{H}, V_{L} and "-" are as defined above.

6. A nucleic acid molecule, **characterized in that** the nucleic acid molecule encodes the chimeric antigen receptor (CAR) as described in claim 1.

7. A vector, **characterized in that** the vector contains the nucleic acid molecule as described in claim 6.

8. A host cell, **characterized in that** the host cell contains the vector as described in claim 7 or a chromosome integrated with an exogenous nucleic acid molecule as described in claim 6 or expressing the CAR as described in claim 1.

9. A formulation, **characterized in that** the formulation contains the chimeric antigen receptor as described in claim 1, the nucleic acid molecule as described in claim 6, the vector as described in claim 7, or the host cell as described in claim 8, and a pharmaceutically acceptable carrier, diluent or excipient.

10. A use of the chimeric antigen receptor as described in claim 1, the nucleic acid molecule as described in claim 6, the vector as described in claim 7, or the host cell as described in claim 8, or the formulation as described in claim 9, which is used for preparing a drug or a preparation for preventing and/or treating cancers or tumors.
